**Europäisches Patentamt**

(19) **European Patent Office**

**Office européen des brevets**

(11) Publication number: **0 124 363**
**B1**

(12) # EUROPEAN PATENT SPECIFICATION

(45) Date of publication of patent specification: **19.12.90**

(51) Int. Cl.⁵: **A 61 L 2/10** // A61K35/16

(21) Application number: **84302845.7**

(22) Date of filing: **27.04.84**

(54) Photochemical decontamination treatment of whole blood or blood components.

(30) Priority: **02.05.83 US 490681**

(43) Date of publication of application:
**07.11.84 Bulletin 84/45**

(45) Publication of the grant of the patent:
**19.12.90 Bulletin 90/51**

(84) Designated Contracting States:
**AT BE CH DE FR GB IT LI LU NL SE**

(56) References cited:
EP-A-0 030 358
EP-A-0 047 462
EP-A-0 066 886
US-A-3 686 395

EXPERIENTIA, vol. 21, no. 1, January 15, 1965,
pages 22-24, Birkhauser Verlag, BASEL (CH). L.
MUSAJO et al.: "Photsensitizing
furocoumarins: interaction with DNA and
photo-inactivation of DNA containing viruses"

(73) Proprietor: **DIAMOND SCIENTIFIC CO.**
**2538 S.E. 43rd Street**
**Des Moines Iowa 50317 (US)**

(72) Inventor: **Wiesehahn, Gary P.**
**1170 9th Street**
**Alameda California (US)**

(74) Representative: **Paget, Hugh Charles Edward**
**et al**
**MEWBURN ELLIS 2 Cursitor Street**
**London EC4A 1BQ (GB)**

(56) References cited:
CHEMICAL ABSTRACTS, vol. 87, no. 11,
September 12, 1977, page 98, abstract no.
78962f, COLUMBUS, OHIO (US). J.E. HEARST et
al.: "The photoinactivation of an RNA animal
virus, vesicular stomatitis virus, with the aid of
newly synthesized psoralen derivatives"

Courier Press, Leamington Spa, England.

**Description**

Recipients of blood and blood components risk acquiring infections from foreign biological organisms, either pre-existing in the blood at the time of collection or transmitted to the blood product during manipulation. Medical personnel who are in contact with collected human blood or clinical samples also have a significant chance of being exposed to potentially lethal blood-borne or sample-borne biological organisms. Blood components today are obtained from blood donors and frequently involve pooled lots, where one or more of the donors may be harboring a viral, bacterial or other infection. Since the blood or blood components are required to provide physiological functions in a mammalian host, normally a human host, these functions must not be impaired by the decontamination treatment of the biological composition. In addition, the blood or blood components may not be modified in such a way as to make them immunogenic which could result in an adverse immune response. Finally, any treatment should not leave residues or products detrimental to the health of the host or such residues or products should be readily removable.

U.S. Patent No. 4,327,086 describes the method for heat treating an aqueous solution containing human blood coagulation factor XIII. U.S. Patent No. 4,321,919 proposes extracorporeal treatment of human blood with 8-methoxypsoralen (8-MOP). Hyde and Hearst, Biochemistry (1978) 17:1251—1257, describe the binding of two psoralen derivatives to DNA and chromatin. Musajo et al., Experientia (1965) XXI, 22—24, describe photo-inactivation of DNA-containing viruses with photosensitizing furocoumarins. U.S. Patent Nos. 4,350,594, 4,348,283 and 4,350,156 describe filtration methods for selective removal of blood components based on molecular weight. U.S. Patent No. 4,329,986 describes extracorporeal treatment of blood with a chemotherapeutic agent which is subsequently removed by dialysis. The July/August 1982 issue of Genetic Engineering News proposed the use of psoralens to sterilize "clinical or commercial reagents or instruments."

Hearst, Nucleic Acids Res. 1977, 4(5) 1339-47 describes the photoinactivation of animal RNA viruses treated with specific psoralen derivatives.

EP—A2—0030358 Leukocyte Research, Inc., describes a method for treating blood to reduce the functioning lymphocyte population, the method involving irradiating blood which has been treated with a psoralen compound.

EP—A2—0066886 Kronenberg, describes a method of inactivating target cells by treating the cells with psoralens and then irradiating them with long wavelength ultraviolet light.

Some data showing substantial impairment of the biological function of certain enzyme proteins using furocoumarins are published in the scientific literature (see for example, Veronese, F. M. et al., Photochem. Photobiol. 34: 351 (1981); Veronese, F. M. et al., Photochem. Photobiol. 36: 25 (1982)).

In the present invention methods and compositions are provided for decontamination of blood components, by permanently inactivating viruses capable of having pathological effect in a mammalian host. Particularly, psoralen compositions are employed for inactivating viral genomes capable of infectious replication in a mammalian host. Aqueous compositions such as blood components may be decontaminated by treatment with psoralen derivatives, and irradiation, preferably with long wavelength ultraviolet (UVA) light, whereby the physiological activities of the non-nucleic acid components are retained.

In decontaminating the blood component(s), an aqueous medium containing the blood component(s) is combined with an appropriate amount of the psoralen composition and irradiated with ultraviolet light under conditions where all of the virus is inactivated, while components other than virus retain their normal physiological activities.

Various blood components may be employed. Red blood cells, blood clotting factors, platelets, plasma (fresh or fresh frozen plasma) and immunoglobulins are of interest. Particular blood components of interest include, plasma protein portion, antihemophilic factor (AHF, Factor VIII); Factor IX and Factor IX complex (Factors II, VII, IX and X); fibrinogens, Factor XIII, prothrombin and thrombin (Factor II and IIa); immunoglobulins (e.g. IgA, IgD, IgE, IgG and IgM and fragments thereof e.g. Fab, F(ab')$_2$, Fc); hyperimmune globulins as used against tetanus and hepatitis B; cryoprecipitate; albumin; interferons; lymphokines, transfer factors; etc. Other biological compositions include vaccines, recombinant DNA produced proteins, oligopeptide ligands, etc. The protein concentration in the aqueous medium will generally range from about 1 µg/ml to 500 mg/ml, more usually from about 1 mg/ml to 100 mg/ml. The pH will normally be close to physiological pH (~7.4), generally in the range of about 6 to 9, more usually about 7. Other components may be present in the medium, such as salts, additives, buffers, stabilizers.

These components will be conventional components, which will be added for specific functions.

The psoralens will include psoralen and derivatives, where the substituents will be: alkyl, particularly of from 1 to 3 carbon atoms, e.g. methyl; alkoxy, particularly of from 1 to 3 carbon atoms, e.g. methoxy; and substituted alkyl, of 1 to 6, more usually 1 to 3 carbon atoms having from 1 to 2 heteroatoms, which will be oxy, particularly hydroxy or alkoxy of from 1 to 3 carbon atoms, e.g. hydroxymethyl and methoxymethyl, or amino, including mono- and dialkyl amino having a total of from 1 to 6 carbon atoms, e.g. aminomethyl. There will be from 1 to 5, usually 2 to 4 substituents, which will normally be at the 4, 5, 8, 4' and 5' positions, particularly at the 4'-position. Illustrative compounds include 5-methoxypsoralen, 8-methoxypsoralen (8-MOP), 4, 5',8-trimethylpsoralen (TMP), 4'-hydroxymethyl-4,5'8-trimethylpsoralen (HMT), 4'-aminomethyl-4,5',8-trimethylpsoralen (AMT), 4-methylpsoralen, 4,4'-dimethylpsoralen, 4,5'-dimethylpsoralen, 4',8-dimethylpsoralen, and 4'-methoxymethyl-4,5',8-trimethylpsoralen.

The subject psoralens are active with a wide variety of viruses and other polynucleotides, DNA or RNA, whether single stranded or double stranded. Illustrative viruses include: adenovirus, arenavirus, bacteriophage, bunyavirus, herpesvirus, orhtomyxovirus, papovavirus, paramyxovirus, picornavirus, poxvirus, reovirus, retrovirus, rhabdovirus, and togavirus. Additional pathogenic microorganisms include bacteria, chlamydia, mycoplasma, protozoa, rickettsia and other unicellular microorganisms. They may also be effective in inactivating Hepatitis B and Non-A Non-B Hepatitis viruses. This inactivation method may also be used against uncharacterized infectious agents which may contain nucleic acid (such as the agent which causes Acquired Immune Deficiency Syndrome).

In addition to the furocoumarins, additives may be included which scavenge for singlet oxygen or other highly reactive oxygen containing species. Such additives include ascorbate, glutathione, sodium thionite, etc. In some instances these additives may have adverse effects, so that in each instance, their use will be determined empirically. Where such additives are present, they will be present in amounts ranging from about 20 µg to 20 mg per ml.

The psoralens may be used individually or in combination, preferably in combination. Each of the furocoumarins may be present in amounts ranging from 0.01 µg/ml to 1 mg/ml, preferably from 0.5 µg/ml to 100 µg/ml, there not being less than 1 µg/ml nor more than 1 mg/ml of psoralens. For RNA, the preferred psoralens are AMT and HMT. For DNA, the preferred psoralens is TMP. For mixtures of DNA- and RNA-containing polynucleotides, or for inactivation of infectious agents or possibly infectious agents of unknown or uncertain nucleic acid classification, or for protection against infections of unknown etiology, preferably TMP and AMT are used in combination.

In carrying out the invention, the psoralens may be added to the biological composition by any convenient means in a manner substantially assuring the uniform distribution in the composition. The composition may then be irradiated under conditions ensuring that the entire composition is exposed to sufficient irradiation, so that the furocoumarins may react with any polynucleotide present to inactivate the polynucleotide. Depending upon the nature of the medium, particularly its opacity, as in the case of blood, the depth of the solution subject to irradiation will vary widely. Usually, the depth will be not less than 0.025 millimeter, but may be a centimeter or more. With whole blood, the depth will generally range from 0.025 millimeter to 2.5 millimeters. The light which is employed will generally have a wavelength in the range of 300 nm to 400 nm. The intensity will generally range from 0.1 mW/cm$^2$ to 5 W/cm$^2$. In order to prevent denaturation, the temperature should be maintained below about 60°C, preferably below about 40°C, usually from about −10°C to 30°C. The medium being irradiated may be irradiated while still, stirred or circulated, and may either be continuously irradiated or be subject to alternating periods of irradiation and non-irradiation. The circulation may be in a closed loop system or it may be in a single pass system ensuring that all of the sample has been exposed to irradiation. The total time for irradiation will vary depending upon the nature of the sample, the furocoumarin derivative used, the intensity and spectral output of the light source and the nature of the polynucleotides which may be present. Usually, the time will be at least 1 min. and not more than about 6 hrs., more usually from about 15 mins. to about 2 hrs. When circulating the solution, the rate of flow will generally be in the range of about 0.1 ml/min to 50 liters/min. It may be desirable to remove the unexpended psoralen and/or its photobreakdown products from the irradiation mixture. This can be readily accomplished by dialysis across an appropriately sized membrane or through an appropriately sized hollow fiber system after completion of the irradiation. It may be desirable in certain applications to remove bound or unbound furocoumarins using antibodies, including monoclonal antibodies, either in solution or attached to a substrate.

The following examples are offered by way of illustration and not by way of limitation.

Experimental

The following experiments were performed in order to demonstrate the ability of the psoralen photoreaction to destroy microbial contaminants contained in whole blood and blood products.

(1) Feline rhinotracheitis virus, a member of the herpesvirus family, was added to heparinized whole rabbit blood in an amount that would give a final concentration of approximately $2 \times 10^7$PFU/ml. 4'-hydroxymethyl-4,5', 8-trimethylpsoralen (HMT) was added to a portion of the rabbit blood and aliquots were irradiated for various periods of time. To test for remaining live virus, duplicate plaque assays were performed using cultured feline cells (Fc3Tg) (ATCC CCL 176), with a methylcellulose overlay. Virus titers were obtained as the arithmetical mean of viral plaques observed in duplicate assay cultures 72 hours after exposure to test samples. The results are as follows:

The blood aliquot that received HMT only and no irradiation gave a titer of $5.3 \times 10^6$PFU/ml. The aliquot that received HMT and five minutes of irradiation exhibited a titer of $4.5 \times 10^5$PFU/ml. In the aliquot that received psoralen plus one hour of irradiation there was no detectable live virus remaining. The sensitivity of this assay should have permitted detection of residual virus at titers $\geq 1.0 \times 10^1$PFU/ml. A blood sample which had received HMT and one hour of irradiation also showed no apparent damage to the red blood cells as judged by phase contrast microscope analysis and by absence of visible hemolysis. These data therefore demonstrate that high virus titers present in whole blood can be inactivated by psoralen plus light treatment which leaves the red cell component of the blood intact.

3

(2) In the second experiment Blue Tongue Virus (Serotype 11), a member of the reovirus family, and Feline Rhinotracheitis Virus, and Simian Virus 40 were added to a solution of Profilate® (a commercial preparation of human clotting factor VIII produced by Alpha Therapeutics). The lyophilized preparation of Profilate® (180 units) was dissolved in 10 ml of sterile water included with the commercial preparation. This solution was further diluted with barbital buffer (11.75 g sodium barbital and 14.67 g NaCl dissolved in 2 liters of de-ionized water and filtered through a 0.22 micron filter) to a final concentration of 5 units per milliliter. One portion (2 ml) was set aside at room temperature in the dark. This was sample #1. A second 2 ml portion was pumped through the apparatus described below for 1 hour with irradiation. This was sample #2. Through addition of appropriate amounts of reagents a third 2 ml portion was adjusted to contain 10 µg/ml AMT and 10 µg/ml HMT and was also irradiated for 1 hour. This was sample #3. The fourth 2 ml portion was adjusted to 10 µg/ml AMT, 10 µg/ml HMT, and 10 mM sodium ascorbate and was also irradiated for 1 hour. This was sample #4. All the samples were kept at 20°C throughout the manipulations. The total elapsed time from dissolving of the lyophilized preparation to the completion of the clotting factor VIII assays was 6 and one-half hours.

The clotting factor VIII assays were performed at a variety of dilutions (ranging from 1:5 to 1:100) for each sample and were compared with the activity in normal human serum and with pooled normal human serum. The results are summarized in Table 1.

TABLE 1

Effect of photochemical inactivation procedure and
its components* on *in vitro* activity of factor VIII[+]

| | | | Sample | | | |
|---|---|---|---|---|---|---|
| Dilution | Normal | Pool | #1<br>F⁻, UVA⁻ | #2<br>F⁻, UVA⁺ | #3<br>F⁺, UVA⁺ | #4<br>F⁺, UVA⁺, ASC⁺ |
| 1:5 | 97 | 108 | 225 | 150 | 186 | 23 |
| 1:10 | 102 | 102 | 245 | 155 | 186 | 28.5 |
| 1:20 | 93 | 92 | 280 | 176 | 196 | 33 |
| 1:50 | 101 | 95 | 265 | 190 | 232 | — |
| 1:100 | — | 100 | 255 | 196 | 263 | — |
| Average | 98 | 99 | 254 | 173 | 213 | 28 |

*F=Furocoumarin;
UVA=long wavelength ultraviolet light;
ASC=sodium ascorbate
[+]Factor VIII activity expressed in % of normal activity.
100%=1U/ml of Factor VIII activity

The sample that was subjected to the psoralen inactivation protocol (sample #3) retained 84% of the factor VIII activity that was present in the control sample (#1). This was higher than the percent activity retained by the sample that was only irradiated (68% retained for sample #2) and indicates that the psoralen photochemistry has little or no effect on the activity of factor VIII.

Surprisingly, the presence of sodium ascorbate (a scavenger of singlet oxygen which has been shown to protect enzymes from inactivation in the presence of psoralens and light) during the irradiation causes the factor VIII activity to drop to 11% of the level in the control sample.

Samples otherwise identical to samples 1, 2, and 3 above were seeded with 2×10⁶PFU/ml of Feline Rhinotracheitis Virus (FeRT), 1×10⁷PFU/ml of Blue Tongue Virus (BTV), and 4×10⁸PFU/ml of Simian Virus 40 (SV-40). Table 2 shows the results of the plaque assays on those samples.

TABLE 2.
Effect of photochemical inactivation
procedure and its components* on infectivity of
virus in factor VIII preparation.+

|  | Sample 1 F⁻, UVA⁻ | Sample 2 F⁻ UVA⁺ | Sample 3 F⁺, UVA⁺ |
|---|---|---|---|
| FeRT titer | $8.6\times10^5$ | $3.5\times10^5$ | 0.0 |
| BTV titer | $3.8\times10^7$ | $1.4\times10^7$ | $1.1\times10^2$ |
| SV-40 titer | $2.5\times10^8$ | $1.6\times10^8$ | $1.2\times10^3$ |

*F=Furocoumarin;
 UVA=long wavelength ultraviolet light.
+Infectivity determined by plaque assays in tissue culture.

In the case of FeRT the number of detectable virus particles was reduced by more than five orders of magnitude to beneath the limit of detection in the plaque assay. The BTV infectivity was reduced by about five orders of magnitude to 110 PFU/ml. The SV40 infectivity was reduced to a titer of $1.2\times10^3$. Thus, it is shown that multiple, widely distinct types of virus can be simultaneously inactivated by at least five orders of magnitude in the presence of factor VIII, using the simple, convenient, brief process described above, with retention of at least 84% of factor VIII activity. Based on the above observations, it is predictable that by extending, repeating or modifying the treatment, the probability of an infectious virus particle remaining can be reduced to an arbitrarily low value. In this manner suitable safety margins can be achieved for any of the cited applications.

Apparatus and system

Since whole blood exhibits very high optical density for longwave UV light (absorption is high for visible light in the 400 nm to 500 nm range), the blood was irradiated through a suitably short optical path length. In this experiment blood was pumped through polyethylene capillary tubing of 0.875 millimeter inside diameter. The tubing was coiled around a 1.27 centimeter diameter tube and immersed in water which was maintained at 18°C. The blood was continuously circulated through the tubing by means of a peristaltic pump. The blood required approximately 2.5 minutes for a complete cycle through the capillary tubing and was in the light beam for approximately 20% of the stated irradiation time. The light source was a low pressure mercury lamp filtered through a cobalt glass filter. The filter transmits light of approximately 320 nm—380 nm, with peak transmittance at 360 nm. The incident intensity at the sample was approximately 40 mW/cm$^2$.

It is evident from the above results, and in accordance with the subject invention, that polynucleotides in biochemical compositions can be inactivated to provide a safe composition for administration to a mammalian host. The proteins present in the composition retain their physiological activity, so that they can fulfill their physiological function in a mammalian host. The method is simple, rapid, and can be expanded to treat large samples. The small amount of chemical reagent required will not generally be harmful to the host.

Although the foregoing invention has been described in some detail by way of illustration and example for purposes of clarity of understanding, it will be obvious that certain changes and modifications may be practiced within the scope of the appended claims.

**Claims**

1. A method for decontaminating blood components suspected of containing viruses, the blood components being red blood cells, platelets, blood clotting factors, plasma or immunoglobulins or a mixture of these, without substantial impairment of the physiological activities of the treated blood components, the method comprising:
   (a) adding to one or more of the blood components at least one psoralen compound; and thereafter
   (b) irradiating said psoralen-treated blood component with long wavelength ultraviolet light under operating conditions which maintain the concentration of reactive oxygen species at levels which do not substantially impair the physiologically active blood component and wherein the amount of the psoralen and the duration of the irradiation is sufficient to inactivate substantially all contaminating viruses present.

2. A method according to Claim 1, wherein at least two psoralen compounds are present.

3. A method according to Claim 1, wherein said blood component is a clotting factor.

4. A method according to any of Claims 1 or 2 wherein any unreacted psoralen(s) or photobreakdown products thereof are selectively removed by ultrafiltration or dialysis.

5. A method according to Claims 1 and 2 wherein psoralen(s) or blood components which have reacted with the psoralen(s) are selectively removed by antibodies to those modified components.

6. A method according to any of the previous claims, said method comprising:

(a) adding to one or more of the blood components at least one psoralen compound in a total psoralen concentration of at least 1 µg/ml and not more than 300 µg/ml; and

(b) passing the psoralen-treated blood component through a light beam with a wavelength in the range of 300 nm to 400 nm at an intensity of 0.1 mW/cm$^2$ to 5 Mw/cm$^2$ at a depth of at least 0.025 mm for a total irradiation time of from 5 min to 12 hrs, the irradiation being conducted under operating conditions which maintain the concentration of reactive oxygen species at levels which do not substantially impair the physiological activity of the treated blood component.

7. A method according to Claim 6, wherein two psoralens are in said medium, said two psoralens being 4'-hydroxymethyl-4,5',8-trimethylpsoralen and 4'-aminomethyl-4,5',8-trimethylpsoralen.

8. A method according to Claim 6, wherein said blood component is a clotting factor.

9. A method according to Claim 1 or Claim 2 wherein Hepatitis A, Hepatitis B and Non-A Non-B Hepatitis viruses are inactivated in the blood components.

10. A method according to Claim 1 or Claim 2 wherein the viral agent which causes Acquired Immune Deficiency Syndrome (AIDS) is inactivated in the blood components.

11. A method according to Claim 1 wherein the level of reactive oxygen containing species is reduced.

12. A method according to Claim 11 wherein the level of reactive oxygen containing species is reduced by the addition of an oxygen scavenger.

**Patentansprüche**

1. Verfahren zum Entkontaminieren von Blutbestandteilen, von denen angenommen wird, daß sie Viren enthalten, wobei die Blutbestandteile rote Blutzellen, Blutplättchen, Blutgerinnungsfaktoren, Plasma, Immunoglobuline oder eine Mischung derselben sind, ohne wesentliche Beeinträchtigung der physiologischen Aktivität der behandelten Blutbestandteile, umfassend:

(a) Versetzen eines oder mehrerer der Blutbestandteile mit wenigstens einer Psoralenverbindung; und darauf

(b) Bestrahlen des genannten, mit Psoralen behandelten Blutbestandteiles mit langwelligem UV-Licht unter Betriebsdbedingungen, unter denen die Konzentration von reaktionsfähigen Sauerstoffgattungen auf Werten gehalten wird, die den physiologisch aktiven Blutbestandteil nicht wesentlich beeinträchtigen und worin die Menge an Psoralen und die Dauer der Bestrahlung ausreicht, um im wesentlichen alle vorliegenden kontaminierenden Viren zu inaktiveren.

2. Verfahren nach Anspruch 1, worin wenigstens zwei Psoralenverbindungen vorliegen.

3. Verfahren nach Anspruch 1, worin der genannte Blutbestandteil ein Gerinnungsfaktor ist.

4. Verfahren nach Anspruch 1 oder 2, worin gegebenenfalls vorliegende(s), nicht umgesetzte(s) Psoralen(e) oder Lichtzersetzungsprodukte des- bzw. derselben selektiv durch Ultrafiltration oder Dialyse entfernt werden.

5. Verfahren nach Anspruch 1 oder 2, worin Psoralen(e) oder Blutbestandteile, die mit dem bzw. den Psoralen(en) reagiert haben, selektiv durch Antikörper für diese modifizierten Verbindungen entfernt werden.

6. Verfahren nach einem der vorhergehenden Ansprüche, umfassend:

(a) Versetzen eines oder mehrerer der Blutbestandteile mit wenigstens einer Psoralenverbindung in einer Psoralengesamtkonzentration von wenigstens 1 µg/ml und nicht mehr als 300 µg/ml; und

(b) Leiten des mit Psoralen behandelten Blutbestandteiles durch einen Lichtstrahl mit einer Wellenläge im Bereich von 300—400 nm und einer Lichtstärke von 0,1 mW/cm$^2$ bis 5 Mw/cm$^2$ bei einer Tiefe von wenigstens 0,025 mm für eine gesamte Bestrahlungszeit von 5 Minuten bis 12 Stunden, wobei die Bestrahlung unter Betriebsbedingungen durchgeführt wird, bei denen die Konzentration an reaktionsfähigen Sauerstoffgattungen auf Werten gehalten wird, die die physiologische Aktivität des behandelten Blutbestandteiles nicht wesentlich beeinträchtigen.

7. Verfahren nach Anspruch 6, worin zwei Psoralene im genannten Medium vorliegen und die beiden Psoralene 4'-Hydroxymethyl-4,5',8-trimethylpsoralen und 4'-Aminomethyl-4,5',8-trimethylpsoralen sind.

8. Verfahren nach Anspruch 5, worin der genannte Blutbestandteil ein Gerinnungsfaktor ist.

9. Verfahren nach Anspruch 1 oder 2, worin Hepatitis A, Hepatitis B und Non-A Non-B Hepatitis-Viren in den Blutbestandteilen inaktiviert werden.

10. Verfahren nach Anspruch 1 oder 2, worin der Viruserreger, der AIDS hervorruft, in den Blutbestandteilen inaktiviert wird.

11. Verfahren nach Anspruch 1, worin der Gehalt an reaktionsfähigen Sauerstoff enthaltenden Gattungen verringert wird.

12. Verfahren nach Anspruch 11, worin der Gehalt an reaktionsfähigen Sauerstoff enthaltenden Gattungen durch Zugabe eines Sauerstoff-Fängers verringert wird.

**Revendications**

1. Méthode de décontamination des composants du sang suspectés de contenir des virus, les composants du sang étant les globules rouges, les plaquettes, les facteurs de coagulation du sang, le plasma ou les immunoglobulines ou un mélange, sans géner sensiblement les activités physiologiques des composants traités du sang, la méthode comprenant:

(a) l'addition à un ou plusieurs des composants du sang d'au moins un composé de psoralène; et en suite

(b) l'irradiation dudit composant du sang traité au psoralène par une lumière ultraviolette de grande longueur d'onde en conditions qui maintiennent la concentration des espèces réactives d'oxygène à des niveaux qui ne génent pas sensiblement le composant physiologiquement actif du sang et où la quantité du psoralène et la durée de l'irradiation est suffisante pour inactiver sensiblement tous les virus contaminants présents.

2. Méthode selon la revendication 1, où au moins deux composés de psoralène sont présents.

3. Méthode selon la revendication 1, où ledit composant du sang est un facteur de coagulation.

4. Méthode selon l'une quelconque des revendications 1 ou 2, où tous les psoralènes n'ayant pas réagi ou leurs produits de photo-décomposition sont sélectivement éliminés par ultrafiltration ou dialyse.

5. Méthode selon les revendications 1 et 2, où le ou les psoralènes ou les composants du sang qui ont réagi avec le ou les psoralènes sont sélectivement éliminés par des anticorps de ces composants modifiés.

6. Méthode selon l'une quelconque des revendications précédentes, ladite méthode comprenant:

(a) l'addition, à un ou plusieurs des composants du sang, d'au moins un composé de psoralène à une concentration totale du psoralène d'au moins 1 µg/ml et pas plus de 300 µg/ml; et

(b) le passage du composant du sang traité au psoralène à travers un faisceau de lumière d'une longueur d'onde comprise entre 300 nm et 400 nm à une intensité de 0,1 mW/cm$^2$ à 5 mW/cm$^2$ à une profondeur d'au moins 0,025 mm, pendant un temps total de rayonnement de 5 minutes à 12 heures, l'irradiation étant entreprise en conditions de fonctionnement qui maintiennent la concentration des espèces réactives d'oxygène à des niveaux qui ne gènent pas sensiblement l'activité physiologique du composant de sang traité.

7. Méthode selon la revendication 6 où deux psoralènes sont dans ledit milieu, lesdits deux psoralènes étant 4'-hydroxyméthyl-4,5',8-triméthylpsoralène et 4'-aminométhyl-4,5',8-triméthylpsoralène.

8. Méthode selon la revendication 6, où ledit composant du sang est un facteur de coagulation.

9. Méthode selon la revendication 1 ou la revendication 2, où les virus de l'Hépatite A, de l'Hépatite B et de l'Hépatite non-A et non-B sont inactivés dans les composants du sang.

10. Méthode selon la revendication 1 ou la revendication 2, où l'agent viral qui provoque le Syndrome Immuno-Déficitaire Acquis (SIDA) est inactivé dans les composants du sang.

11. Méthode selon la revendication 1, où le niveau des espèces contenant de l'oxygène réactif est réduit.

12. Méthode selon la revendication 11, où le niveau des espèces contenant de l'oxygène réactif est réduit par addition d'un épurateur d'oxygène.